# EUROPÄISCHE PATENTSCHRIFT

(11) **EP 0 873 427 B1**
(45) Veröffentlichungstag und Bekanntmachung des Hinweises auf die Patenterteilung: **28.08.2002**
(21) Anmeldenummer: 97906982.0
(22) Anmeldetag: 07.01.1997
(51) Int. Cl.: C12Q 1/68, G01N 33/53

(54) **VERFAHREN ZUR ERFASSUNG UND QUANTIFIZIERUNG VON NUCLEINSÄURE-MOLEKÜLEN**
PROCESS FOR DETECTING AND QUANTIFYING NUCLEIC ACID MOLECULES
PROCEDE DE DETECTION ET DE QUANTIFICATION DE MOLECULES D'ACIDE NUCLEIQUE

(30) Priorität: 08.01.1996 DE 19600362
(43) Veröffentlichungstag der Anmeldung: 28.10.1998
(73) Patentinhaber: november Aktiengesellschaft Gesellschaft für Molekulare Medizin, 91056 Erlangen (DE)
(72) Erfinder: november Aktiengesellschaft Gesellschaft für Molekulare Medizin, 91056 Erlangen (DE)
(74) Vertreter: Gassner, Wolfgang, Dr.
(86) Internationale Anmeldenummer: DE9700008
(87) Internationale Veröffentlichungsnummer: WO97025441

(56) Entgegenhaltungen:
- WO-A-93/22457
- WO-A-95/06652
- WO-A-95/07361
- GB-A- 2 179 735
- NUCLEIC ACIDS RESEARCH, Bd. 18, Nr. 13, 1990, Seiten 3933-39, XP002033171 GANGULY, A ET AL: "Detection of single-base mutations by reaction of heteroduplexes with a water-soluble carbodiimide followed by primer extension: application to products from the polymerase chain reaction" in der Anmeldung erwähnt
- MUTATION RESEARCH, Bd. 288, Nr. 1, Juli 1993, Seiten 65-77, XP000602208 SMOOKER P M ET AL: "THE USE OF CHEMICAL REAGENTS IN THE DETECTION OF DNA MUTATIONS"
- DEVELOPMENTS IN BIOLOGICAL STANDARDIZATION, Bd. 86, November 1994, Seite 325 XP000676820 AMEXIS G ET AL: "Detection of a point mutation in poliovaccine with a water soluble carbodiimide reaction of DNA heteroduplexes"
- NATURE GENETICS, Bd. 9, 1.Februar 1995, Seite 103/104 XP000600254 DEAN M: "RESOLVING DNA MUTATIONS"
- NATURE GENETICS, Bd. 9, 1.Februar 1995, Seiten 177-183, XP000600255 MASHAL R D ET AL: "DETECTION OF MUTATIONS BY CLEAVAGE OF DNA HETERODUPLEXES WITH BACTERIOPHAGE RESOLVASES"
- NUCLEIC ACIDS RESEARCH, Bd. 23, Nr. 24, 1995, Seiten 5082-5084, XP000606029 BABON J J ET AL: "IMPROVED STRATEGY FOR MUTATION DETECTION--A MODIFICATION TO THE ENZYME MISMATCH CLEAVAGE METHOD"

## Beschreibung

Die Erfindung betrifft ein Verfahren nach dem Oberbegriff des Anspruchs 1.

Ein wichtiges Gebiet der Molekularbiologie betrifft die Erfassung von Sequenzabweichungen in Mischungen weitgehend homologer Nucleinsäuren (= NA). Der Sequenzvergleich zwischen DNA-Molekülen zur Identifizierung von Abweichungen trägt nicht nur zum Wissen über die molekularen Grundlagen phänotypischer Variationen, bsp. Erbkrankheiten, bei, sondern erlaubt auch eine fortlaufende Überwachung von NA-Populationen, bsp. Viruspopulationen, während einer Infektion. Dabei wird unter einer NA-Population eine Mehrzahl an NA-Molekülen identischer, gleicher oder unterschiedlicher Sequenz verstanden. Des weiteren dient der Sequenzvergleich auch als Qualitätssicherungsmerkmal bei der Herstellung gentechnologischer, bakterieller oder viraler Produkte oder zum Erkennen des Auftretens sehr kleiner Mengen abweichender Sequenzen in einer Population homologer Sequenzen.

Nach dem Stand der Technik sind mehrere Verfahren zum Aufspüren von Sequenzabweichungen bekannt. Das wohl mühsamste Verfahren ist das direkte Sequenzieren (Sanger F., Nicklen S., Coulson A.R. 1977 Proc. Natl. Acad. Sci. USA 74, 5463 ff; Maxam A.M., Gilbert W. 1977 Proc. Natl. Acad. Sci. USA 74, 560-564). Dieses Verfahren erlaubt es nicht, statistisch signifikante Anzahlen von Individuen einer NA-Population auf das Auftreten von Mutationen hin zu untersuchen. - Auch die Anwendung indirekter Hybridisationsmethoden wie Southern (Southern E.M. 1975 J. Mol. Biol. 98, 503-517) oder Northern (Alwine J.C., Kemp D.J., Stark G.R. 1977 Proc. Natl. Acad. Sci. USA 74, 5350-5354) erlauben nur die Detektion massiver quantitativer Abweichungen. - Etwas sensitiver sind Verfahren wie die Ribonuclease-Protektionsassays (D.J. Freeman, A.S. Juan 1981 J. Gen. Virol. 57, 103-117; E. Winter et al. 1985 PNAS 82, 7575-7579) für Ribonucleinsäure(=RNA)-RNA Heteroduplices oder für RNA-Desoxyribonucleinsäure(=DNA) Heteroduplices (R.M. Myers et al., 1985 Science 230, 1242-1246).

Die denaturierende Gradientengelelektrophorese (DGGE) ist in den letzten Jahren durch den Einsatz der "polymerase chain reaction"(=PCR)-Technologie sowie die Verwendung von speziellen Primern, die eine Auftrennung im Gradientengel erleichterten (V.C. Sheffield et al., 1992 Biofedback 12, 386-387), deutlich sensitiver gemacht worden. Zur Auftrennung der Reaktionsprodukte müssen auch dabei die abweichenden Sequenzen in deutlichen Mengen vorliegen. Ein weiterer Nachteil dieses Verfahrens besteht darin, daß nach der Auftrennung und Detektion einer Mutante keine Aussage über den Ort der Mutation gemacht werden kann, so daß nachfolgend weitere Identifizierungsreaktionen, bsp. eine Sequenzierung, notwendig sind.

*"Chemical cleavage reactions"* unter Verwendung von Hydroxylamin und Osmiumtetroxid haben den Nachteil, daß viele experimentelle Manipulationen mit toxischen Chemikalien und komplexe Arbeitsabläufe (R.G.H. Cotton, 1989 Biochemistry 263, 1-10) erforderlich sind. Darüber hinaus funktionieren auch sie nur, wenn größere Mengen von Mutanten vorliegen. Schließlich können mit diesem Verfahren nur bestimmte Mutationen identifiziert werden.

Ein weiteres Verfahren nach dem Stand der Technik beruht auf der *"single Strand conformation polymorphism"* (SSCP)- Reaktion. Diese Methode wird ebenso wie die DGGE durchgeführt (M. Urita et al., 1989 PNAS 86, 2766-2770). Ein Nachteil der SSCP-Reaktion besteht darin, daß sie zur Identifizierung von falsch-positiven Proben führt. Des weiteren versagt dieses Verfahren in wenigstens 10% aller Fälle, wenn große Mengen an mutanten Molekülen vorliegen.

Weitere Verfahren nach dem Stand der Technik erlauben lediglich das Überprüfen des Vorliegens einer bestimmten Mutation, d.h. die Verifizierung der An- bzw. Abwesenheit eines einzelnen Nucleotids (MAPREC: Chumakov K.M., Powers L.B., Noonan K.F., Roninson L.B., Levenbook I.S. 1991 Proc. Natl. Acad. Sci. USA 88, 199-203).

Verfahren unter Verwendung von Carbodiimid haben sich bisher aufgrund der schweren Handhabbarkeit dieses Stoffs und ihrer mangelnden Sensitivität in der Praxis nicht durchsetzen können (D.F. Novack, 1986 Proc. Natl. Acad. Sci. USA, 83, 586-590; A. Ganguly, 1991 J. Biol. Chem. 266, 1235-1240; Offenlegungsschrift DE 36 29 190 AI, A. Ganguly und D.J. Prockop, 1993 Nucl. Acids Res. 18 No.13, 3933-3939).

Ein weiteres nach dem Stand der Technik bekanntes Verfahren (WO 93/02216) ist das Verfahren zur Erkennung von "mismatch" in Heteroduplexen. Dabei wird ein "mismatch-bindendes Protein" verwendet, das von ersten Antikörpern gebunden wird. Diese ersten Antikörper werden wiederum von zweiten Antikörpern erkannt. Auch dieses Verfahren ist schwer durchführbar und nur begrenzt einsetzbar.

Aus Nuc.Ac.Res. 18/13, 1990, Seiten 3933 bis 3939, ist ein Verfahren zur Detektion einer Untergruppe von Nukleinsäuremolekülen in einem Nukleinsäuregemisch bekannt. Sofern eine große Menge an mutanten Molekülen vorliegt, eignet sich das bekannte Verfahren nicht zur Quantifizierung erfaßter NA-Moleküle.

Insgesamt stellt die mangelnde Sensitivität bezüglich der Mindestmenge an vorliegenden Mutanten innerhalb einer NA-Population den Hauptnachteil bei den nach dem Stand der Technik bekannten Verfahren dar. Auch ist eine Quantifizierung der erfaßten NA-Moleküle nicht möglich. Ein weiters Problem der bekannten Verfahren ist deren zu hohe Versagensrate.

Der vorliegenden Erfindung liegt die Aufgabe zugrunde, ein Verfahren bereitzustellen, das die Nachteile nach dem Stand der Technik beseitigt.

Die Aufgabe wird durch die Merkmale des Patentanspruchs 1 gelöst. Zweckmäßige Weiterbildungen ergeben sich aus den Merkmalen der Patentansprüche 2 - 15.

Das erfindungsgemäße Verfahren hat den Vorteil, daß es insbesondere auf unamplifizierte genomische DNA angewendet werden kann. Ein weiterer bemerkenswerter Vorteil besteht darin, daß in einer einzigen Stufe der Reaktion die Mutation innerhalb des Fragmentes zu lokalisiert werden kann. Auch können geringe Mengen an mutanten Nukleinsäuren in einer Population homologer Moleküle detektiert werden. Bis zu einem Anteil von 50 % Mutanten führt das immer zu einer Vermehrung der in der Population vorliegenden sequenzmäßig abweichenden Moleküle. Das erfindungsgemäße Verfahren kann daher bsp. zur Sekundäranalyse amplifizierter DNA verwendet werden. Auf diese Weise kann erkannt werden, ob eine erfaßte und identifizierte Sequenz typisch für die vorliegende NA-Population ist oder ob sie auf eine Kontamination mit ähnlicher DNA zurückzuführen ist. So können auch Kontaminationen im molekularen Maßstab erkannt werden, die bei Anwendung von PCR-Verfahren zu einer erheblichen Verfälschung der Nachweisreaktionen führen. Das erfindungsgemäße Verfahren eröffnet somit gerade in Kombination mit PCR-Verfahren drastisch verbesserte Nachweisergebnisse.

Das Gemisch von homo- und heteroduplexen DNA-Molekülen kann mit der Carbodiimidverbindung zu Carbodiimidumsatzprodukten umgesetzt werden. Vorteilhafterweise ist dabei vorgesehen, daß das Gemisch von homo- und heteroduplexen DNA-Molekülen zur Erkennung einer mangelnden Basenpaarung mit NA-spezifischen Enzymen und Co-Faktoren versetzt wird. Eine weitere besonders vorteilhafte Ausgestaltung des Verfahrens besteht darin, daß das mit der Carbodiimidverbindung umgesetzte Gemisch von homo- und heteroduplexen DNA-Molekülen, vorzugsweise nach einer Aufreinigung, einer Extensionsreaktion, insbesondere einer Primerextensionsreaktion, unterworfen wird. Die Produkte der Extensionsreaktion können schließlich charakterisiert und quantifiziert werden. Dabei kommen gängige Verfahren, bsp. PCR-Verfahren, zum Einsatz.

Besonders genau ist das erfindungsgemäße Verfahren dann, wenn die Extensionsreaktion mehrfach durchgeführt wird. Der Primer oder die Extensionsprodukte sind vorzugsweise markiert. Als besonders vorteilhaft wird angesehen, markierte Oligonucleotide zu verwenden. Die Markierung der Oligonucleotide kann in einem separaten Schritt durchgeführt werden. Das ermöglicht es, die zu inkorporierenden Markierungsmittel abzutrennen. Insbesondere durch die Verwendung von großen- und identitätsselektionierten Oligonucleotiden kann eine Erhöhung der Spezifität des Verfahrens erreicht werden.

Besonders sensitiv ist das Verfahren, wenn der Aufreinigungsschritt ein chromatographisches Aufreinigungsverfahren umfaßt. Das chromatographische Aufreinigungsverfahren kann ein Säulen- oder batch-Verfahren sein, das unter Verwendung von Matrices wie Silikagel oder DEAE-Material durchgeführt wird, welche eine Trennung nach dem Prinzip des Ionentauschers, der Affinität oder des Größenausschlusses ermöglichen. Der Aufreinigungsschritt kann insbesondere unter Verwendung einer Silika-Säule durchgeführt werden. Das hat den Vorteil, daß auf eine thermische Zerstörung von Carbodiimidresten sowie auf die nach dem Stand der Technik erforderliche Extraktion und Fällung unter Verwendung von Ethanol verzichtet werden kann.

Nachfolgend wird zunächst allgemein die Durchführung des erfindungsgemäßen Verfahrens am Beispiel der Mutationsdetektion bei NA-Populationen näher erläutert.

Die Vorbereitung des Heteroduplexes für die Umsetzung mit Carbodiimid beinhaltet eine Amplifikationsreaktion mit geeigneten Primern und, im Falle von RNA, einer vorausgehenden Generierung von cDNA in einer reversen Transkriptionsreaktion. Die Detektion der Reaktionsprodukte kann über Gele, bevorzugterweise hochprozentige Polyacrylamidgele, aber auch durch andere chromatographische Methoden, wie bsp. Silika-Säulen bzw. Silikagelsäulen, HPLC(= high pressure liquid chromatography) oder mittels Antikörpern, durchgeführt werden.

Bei dem erfindungsgemäßen Verfahren können vor oder während der Behandlung mit Carbodiimid noch weitere "mismatch erkennende Substanzen", wie z.B. die Proteine Mut-L oder Mut-S, zur Verstärkung der Reaktionsfähigkeit der Carbodiimidderivate eingesetzt werden. Hat eine reverse Transkription und/oder eine Amplifikation der Individuen der Population stattgefunden, so ist es günstig, für die darauffolgende enzymatische Reaktion mit DNA-Polymerase Primer zu wählen, die nicht außerhalb der für die Amplifikation bzw. reversen Transkription liegenden Primer liegen. Im Falle der Analyse von Homodimeren im Anschluß an eine Amplifikation (mittels PCR) ergibt sich ein Primerextensionsprodukt von definierter Länge. Das Vorliegen einer Mutation bedingt die Bildung eines Heteroduplexes, was zur Entstehung eines carbodiimidmodifizierten Nucleotids an einer bestimmten Stelle führt und den Abbruch der Replikation der Kette durch die DNA-Polymerase bewirkt. Je nach Lage der Mutation entsteht so ein Abbruchprodukt von charakteristischer Länge, das in einer nachfolgenden Analyse auf sein Vorhandensein (bsp. Antikörper gegen carbodiimidmodifizierte Nucleotide) oder seine Länge (mittels Gelelektrophorese oder chromatographischen Methoden, bsp. HPLC) identifiziert werden kann.

Die Analyse einer RNA-Population kann durch ein Analyseschema mit den folgenden Schritten beschrieben werden:
- Durch einen geeigneten Primer wird in einer reversen Transkriptionsreaktion eine einzelsträngige oder auch doppelsträngige DNA für die weiteren Schritte bereitgestellt, hergestellt, gewonnen oder anderweitig erworben. Bei der Herstellung aus RNA kann es günstig sein, ein Enzym zu verwenden, das bei höheren Temperaturen arbeiten kann. In einem anschließenden Schritt wird mit Hilfe eines geeigneten Primers aus einer eventuell vorliegenden einzelsträngigen DNA doppelsträngige DNA hergestellt, gewonnen oder anderweitig erworben.
- Doppelsträngige DNA-Moleküle können, müssen jedoch nicht, einer Amplifikationsreaktion unterworfen werden. Dabei kann es sich um die als PCR bekannte Reaktion oder eine Abart davon, bsp. die polymerase chain ligation, handeln.
- Nach Abschluß dieser vorbereitenden Arbeiten liegen doppelsträngige homodimere DNA-Moleküle vor. Das Verhältnis der Homodimeren entspricht auch im Falle einer vorangegangenen Amplifikation immer noch dem in der ursprünglichen Population (I) vorherrschenden Verhältnis der einzelnen Sequenzvarianten. In einem folgenden Denaturierungsschritt, der bevorzugterweise in einer geeigneten Lösung durch Temperaturerhöhung herbeigeführt wird, werden die homodimeren Doppelstränge aufgeschmolzen. In einem anschließenden Renaturierungsschritt, der vorzugsweise durch Temperaturerniedrigung ausgelöst wird, verbinden sich die homologen Einzelstränge statistisch zu Homo- und Heteroduplexen. Dabei ist zu beachten, daß mit fallendem Anteil an sequenzabweichenden Individuen in der ursprünglichen Population (I) die Wahrscheinlichkeit steigt, daß jeder der mutanten Einzelstränge zu einer Bildung eines Heteroduplexes führt. Im Extremfall ist dabei die Zahl der gebildeten Heteroduplexe doppelt so groß wie die Zahl der Individuen, welche in der ursprünglichen Population (I) eine abweichende Sequenz aufgewiesen haben.
- Im nächsten Schritt erfolgt in An- oder Abwesenheit mutationsdetektierender Proteine die Umsetzung der heteroduplexen homodimeren Gemische mit Carbodiimidderivaten. Bei Wahl geeigneter Konzentrations- und Pufferbedingungen findet keine Kopplung von Carbodiimidderivaten an Homodimere statt.
- Danach werden die Nucleinsäuren und Carbodiimidderivaten von den Umsetzungsprodukten der NA, vorzugsweise über eine Säule, bsp. eine Silikagel-Säule, voneinander getrennt. Die so gereinigten und ggf. in weiteren Schritten unkonzentrierten Umsetzungsprodukte stehen nun für die Verwendung in einer anschließenden enzymatischen Reaktion zur Verfügung.
- In einem geeigneten Puffermilieu unter Zusatz von Nucleotiden, die als Triphosphate markiert sein können, und geeigneten Primern, die radioaktiv markiert sein können, wird der Mischung eine DNA-Polymerase zugesetzt, die anhand der an Matritzen-Nucleinsäurevorlagen gebundenen Primer Replikate dieser Vorlagen herstellen. Das Vorliegen einer Carbodiimidmodifikation führt zum Abbruch der Kettenverlängerung des frisch replizierten NA-Moleküls und dadurch zum Vorliegen eines vorzugsweise markierten Reaktionsprodukts, das eine für die jeweilige Lokalisierung der Sequenzabweichung charakteristische Länge vorweist. - Eine Erhöhung der Sensitivität kann durch die Verwendung radioaktiv markierter Oligonucleotide erreicht werden.
- In einer abschließenden Reaktion oder Folge von Reaktionen kann dieses Replikationsprodukt charakterisiert werden. Vorzugsweise wird der Reaktionsansatz auf einem hochprozentigen Polyacrylamidgel aufgetragen und aufgetrennt, was eine gleichzeitige Bestimmung der Länge und der Quantität des Ausgangs- und des mutanten NA-Populationsanteiles erlaubt. Eine Auftrennung und Quantifizierung des Reaktionsprodukts läßt sich jedoch auch durch andere chromatographische Methoden, bsp. den Einsatz einer HPLC-Analyse, bewerkstelligen. Unter Umständen mag es auch sinnvoll sein, das Reaktionsprodukt mit Antikörpern, die spezifisch für mit Carbodiimid modifizierte Nucleotide sind, umzusetzen und für eine weitere Detektion vorzubereiten. Das Binden von Antikörpern mag zwar primär eine Größenbestimmung des Reaktionsprodukts erschweren, kann aber, speziell im Zusammenhang mit weiteren antikörperspezifischen Antikörpern, zu einer Erhöhung der Sensitivität führen.

Nachfolgend werden zwei Ausführungsbeispiele zur Durchführung des erfindungsgemäßen Verfahrens beschrieben:

### Beispiel 1:

Die Umsetzung von Heteroduplices mit Carbodiimid

### a.) Generierung von DNA Heteroduplices:

Für einen Reaktionsansatz werden zwei Nucleinsäuren verwendet, welche dieselbe Länge haben und sich in ihrer Sequenz nur an einer Stelle unterscheiden. Bei der Sequenz handelt es sich um den Abschnitt von Nucleotid Nr. 394 bis zum Nucleotid 572 der attenuierten Poliomyelitisvirus Serotyp 3. Der Sequenzunterschied zwischen den beiden eingesetzten Nucleinsäuren liegt an der Position 472. Etwa 99 % der NA-Population tragen an dieser Stelle ein Thymin als Base, 1 % trägt an dieser Stelle ein Cytosin als Base jeweils auf dem codierenden Strang. Beide NA-Varianten liegen doppelsträngig vor und werden durch eine konventionelle Amplifikationsreaktion, nämlich durch das im EP-A-0200362 beschriebene PCR-Verfahren, vermehrt. Die wäßrige Phase der PCR wird nach der Reaktion mit dem fünffachen Volumen an PB-Puffer gemischt und anschließend für 1 min. bei 15000 Umdrehungen pro Minute (=rpm) durch eine Silikagelsäule zentrifugiert. Die Silikagelsäule wird im nächsten Schritt mit 750 µl Guanidiniumchlorid-Lösung (35 g auf 100 ml Wasser) gewaschen und erneut 1 min. bei 15000 rpm zentrifugiert. Dadurch werden die restlichen Primer und Dimere von der Silikagelsäule entfernt. Anschließend werden 750 µl PE Waschpuffer auf die Silikagelsäule aufgetragen und ebenfalls für 1 min. zentrifugiert. Die Reste des Waschpuffers in der Silikagelsäule werden durch erneutes Zentrifugieren für 1 min. entfernt. Die Silikagelsäule wird darauf in ein 1,5 ml Reaktionsgefäß überführt und die auf der Silikagelsäule gebundene DNA in 50 µl Wasser durch Auftragen und erneutes Zentrifugieren für 1 min. eluiert.

### b) Carbodiimid-Modifikation der DNA Heteroduplices:

20 µl des Eluats werden in ein silikonisiertes "thin-walled tube" überführt. Die nun vorliegende Menge an DNA variiert zwischen 40 und 500 ng. Normalerweise beträgt die Menge dabei 200 ng pro Ansatz. Nach Zugabe von 10 µl Hybridisierungspuffer (3 M Natriumchlorid; 35 mM MgCl₂ in 30 mM Tris-HCl-Puffer, pH 7,4) und 70 µl Wasser wird der Ansatz mit 2 Tropfen Mineralöl überschichtet und 10 min. bei 100°C im Wasserbad denaturiert, bevor er unmittelbar danach auf Eis gegeben wird. Die Annealing-Reaktion findet über Nacht bei 42°C statt. Danach wird das fünffache Volumen an PB-Puffer zugegeben (enthält Guanidiniumhydrochlorid) gemischt und 1 min. zentrifugiert. Es folgt eine erneute Aufreinigung über die Silikagelsäule. Die Elution erfolgt in 60 µl TE (0,1 mM EDTA, 10 mM Tris-HCl, pH 7,4). Eine frische 200 mM Carbodiimid-Lösung (84,7 mg/ml) wird unmittelbar vorher angesetzt (CME-Carbodiimid: N-Cyclohexyl-N-(2-morpholinoethyl)-carbodiimidmethyl-p-toluolsulfonat). Die Carbodiimid-Lösung wird mit der gewünschten Menge Heteroduplex DNA (20 - 200 ng), die durch die Annealing-Reaktion entstanden ist, versetzt. Nach Zugabe von 4 µl 1 M Natriumborat-Lösung, pH 8,0, werden 10 µl der Carbodiimid-Lösung zugegeben und 3 h bei 30°C inkubiert. Anschließend wird der Ansatz auf 40 µl Wasser ergänzt.

### c) Entfernen von nicht gebundenem Carbodiimid aus dem Reaktionsgemisch:

Der Carbodiimid-Modifikationsansatz wird mit dem fünffachen Volumen an PB-Puffer versetzt, gemischt und 1 min. zentrifugiert. Es folgt ein erneuter Säulenreinigungs-Schritt, wobei der Waschvorgang dreimal wiederholt wird. Die DNA wird von der Silikagelsäule mit 26 µl TE (pH 7.4) über Nacht bei Raumtemperatur eluiert. Anschließend wird die Silikagelsäule 1 min. zentrifugiert.

### Beispiel 2:

### Für Carbodiimid-Konjugate spezifische Primerextension:

Ein typischer Primer-Extensionsansatz besteht aus 5 µl der im Beispiel 1 beschriebenen Heteroduplex-DNA und 5 µl des PCR-Gemischs, der die Nucleotide A, G und T in Konzentration von 0,4 mM, das Nucleotid C in einer Konzentration von 20 µM enthält und ferner 100 µCi an p32 dCTP. Neben 100 pmol des für die Primerextension benötigten Oligonucleotids und 5 units der Taq DNA Polymerase enthält der 100 µl Ansatz noch Puffer (10 mM Tris-HCl, pH 8,3, 3,5 mM MgCl₂; 75 mM KCl) und BSA in einer Endkonzentration von 7 µg/ml. Die Proben werden erst in den Perkin Eimer Cycler 480 gegeben, wenn die Temperatur auf 90°C angestiegen ist. Die Reaktion ist nach einem durchlaufenen Zyklus beendet. Danach werden die Proben auf Eis gelagert. Der Zyklus besteht aus einer Denaturierung (70 sec. bei *96°C*), gefolgt von einer Annealingreaktion (30 sec. bei 62°C) und einer Extension (1 min. bei 72°C). Nach Ende der Reaktion werden 5 µl Auftragspuffer (50% Sucrose, 0,1 M EDTA pH 8,0, 0,1 % Bromphenolblau, 0,1 % Xylenxylanol) zugegeben und der Ansatz auf Eis gelagert. Danach wird ein 8 µl Aliquot auf ein 10%-iges PAA-Gel auf getragen. Die Detektion kann durch Auflegen eines Röntgenfilms geschehen oder durch Exposition in einem Phosphorimager, was die Quantifizierung erleichtert.

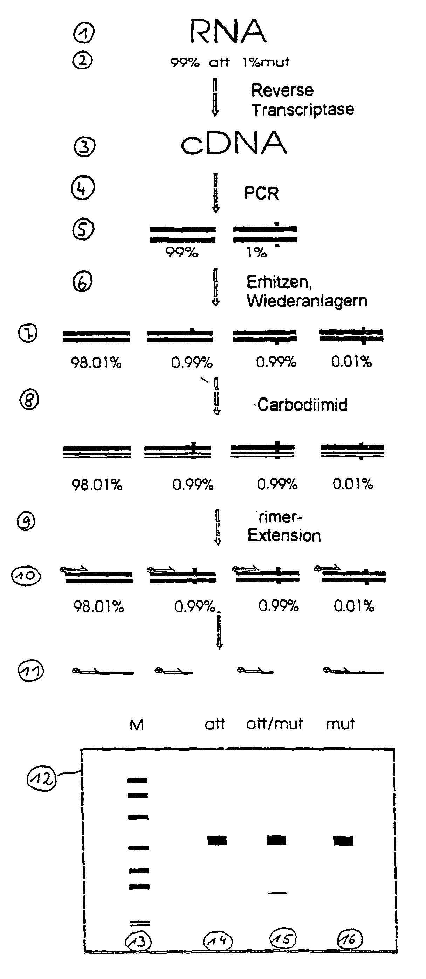

Das in den vorhergehenden Beispielen beschriebene Verfahren ist in Form eines Flußdiagramms in der Zeichnung dargestellt:

Als Ausgangspopulation wurde eine RNA-Population als Beispiel auswählt, die 99 % attenuierte(=att) und 1 % mutierter(=mut) RNA-Viren repräsentiert (Bezugszeichen 1). Nach einer reversen Transkription 2 entsteht im gleichen Anteil cDNA 3. In einem weiteren Schritt wird diese cDNA durch eine PCR-Reaktion 4 über 30 Zyklen amplifiziert. Das Verhältnis attenuierter zu mutierter Amplifikate beträgt immer noch 99:1% (Bezugszeichen 5). In einem letzten Denaturierungs- /Renaturierungsschritt 6, wobei eine Aktivität einer DNA-Polymerase ausgeschlossen wurde, liegen Homo- und Heteroduplexe 7 in nunmehr neuen Verhältnissen vor. Dieses Gemisch an Homo- und Heteroduplexen 7 wird nun mit Carbodiimid 8 umgesetzt und nach Aufreinigung einer Primer-Extensionsreaktion 9 unterworfen. Anschließend erfolgt der Markerauftrag 10. Die markierten Reaktionsprodukte 11 werden schließlich durch Gelelektrophorese 12 analysiert. Ein schematisches Ergebnisbild zeigt neben einem Marker M die Analyse eines reinen homoduplexen Ansatzes, eines gemischten hetero- homoduplexen Ansatzes und eines reinen heteroduplexen Ansatzes. Das Bezugszeichen 13 bezeichnet den Markerauftrag, Bezugszeichen 14 eine erste Kontrollspur 100%ig attenuierter NA; das Bezugszeichen 15 den Analysenauftrag und Bezugszeichen 16 eine zweite Kontrollspur 100%ig mutierter NA.

## Patentansprüche

1. Verfahren zur Erfassung und Quantifizierung von Nucleinsäure(=NA)-Molekülen innerhalb einer Population (I) von NA-Molekülen identischer, ähnlicher oder abweichender Sequenz, bei dem
a) die in der Population (I) enthaltenen einzel- oder doppelsträngigen Ribonucleinsäure(=RNA)-Moleküle oder einzelsträngigen Desoxyribonucleinsäure(=DNA)-Moleküle in eine Population (II) doppelsträngiger DNA-Moleküle überführt werden,
b) die Population (II) doppelsträngiger DNA-Moleküle einer Denaturierung und anschließenden Renaturierung unterworfen wird, so daß ein Gemisch von homo- und heteroduplexen DNA-Molekülen vorliegt
c) in der Population (II) gebildete Heteroduplexe, bei denen ungepaarte oder nicht korrekt nach Watson und Crick gepaarte Nucleotide vorliegen, chemisch mit einer Carbodiimidverbindung umgesetzt werden sowie
d) die entstandenen Carbodiimidumsetzungsprodukte charakterisiert werden,
**dadurch gekennzeichnet, daß**
zwischen jedem der Schritte lit. a bis lit. d ein chromatographisches Aufreinigungsverfahren durchgeführt wird.

2. Verfahren nach Anspruch 1, **dadurch gekennzeichnet, daß** das chromatographische Aufreinigungsverfahren ein Säulenoder batch-Verfahren ist, das unter Verwendung von Silikagel durchgeführt wird.

3. Verfahren nach einem der vorhergehenden Ansprüche, **dadurch gekennzeichnet, daß** der Aufreinigungsschritt unter Verwendung einer Silika-Säule durchgeführt wird.

4. Verfahren nach einem der vorhergehenden Ansprüche, **dadurch gekennzeichnet, daß** als Cabodiimidverbindung eine oder mehrere der folgenden Verbindungen verwendet werden: N-Cyclohexyl-N'-(2-morpholinoethyl)-carbodiimid; Adipindiimidsäuredimethylester; Pyromellitsäurediimid; N,N'-Bis(2,6-dimethylphenyl)perylen-3,4,9,10-tetracarbonsäurediimid; Bis-(trimethylsilyl)-carbodiimid; N,N'-Ditertiärbuylcarbodiimid; N,N'-Dicyclohexylcarbodiimid; N,N'-Diisopropylcarbodiimid; N-(3-Dimethylaminopropyl)-N'-ethylcarbodiimid; N,N'-Ditridecylperylen-3,4,9,10-tetracarbonsäurediimid; Pimelindiimidsäuredimethylester, Octandiimidsäuredimethylester; 1-Ethyl-3-(3-dimethylaminopropylcarbodiimid

5. Verfahren nach einem der vorhergehenden Ansprüche, **dadurch gekennzeichnet, daß** das Gemisch von homo- und heteroduplexen DNA-Molekülen zur Erkennung einer mangelnden Basenpaarung mit NA-spezifischen Enzymen und Co-Faktoren versetzt wird.

6. Verfahren nach Anspruch 1, **dadurch gekennzeichnet, daß** das Gemisch von homo- und heteroduplexen DNA-Molekülen mit der Carbodiimidverbindung, vorzugsweise unter Zusatz von "mismatch-erkennenden Proteinen", umgesetzt wird.

7. Verfahren nach Anspruch 1 oder 2, **dadurch gekennzeichnet, daß** die Population (II) vor dem Schritt lit. c einem oder mehreren Amplifikationschritt/en unterworfen wird.

8. Verfahren nach einem der Ansprüche 4 bis 7, **dadurch gekennzeichnet, daß** im Schritt lit. d das mit der Carbodiimid-Verbindung umgesetzte Gemisch von homo- und heteroduplexen DNA-Molekülen einer Extensionsreaktion unterworfen und die Extensionsprodukte charakterisiert und quantifiziert werden.

9. Verfahren nach einem der vorhergehenden Ansprüche, **dadurch gekennzeichnet, daß** die Extensionsreaktion ohne Primer durchgeführt wird.

10. Verfahren nach Anspruch 8, **dadurch gekennzeichnet, daß** eine Primerextensionsreaktion durchgeführt wird.

11. Verfahren nach einem der vorhergehenden Ansprüche, **dadurch gekennzeichnet, daß** zur Extensionsreaktion ein Oligonucleotid in modifizierter oder nicht modifizierter Form verwendet wird.

12. Verfahren nach einem der vorhergehenden Ansprüche, **dadurch gekennzeichnet, daß** der Primer oder die Extensionsprodukte markiert sind oder werden.

13. Verfahren nach einem der vorhergehenden Ansprüche, **dadurch gekennzeichnet, daß** die Extensionsreaktion mehrfach durchgeführt wird.

14. Verfahren nach einem der vorhergehenden Ansprüche, **dadurch gekennzeichnet, daß** eine Detektion von Carbodiimidumsatzprodukten ohne vorherige Extensionsreaktion durchgeführt wird.

15. Verfahren nach einem der vorhergehenden Ansprüche, **dadurch gekennzeichnet, daß** die Carbodiimidumsatzprodukte mittels carbodiimidspezifischer Antikörper erkannt werden.

## Claims

1. A process for detecting and quantifying nucleic acid (=NA) molecules within a population (I) of NA molecules of an identical, similar of different sequence, in which
a) the single-stranded or double-stranded ribonucleic acid (=RNA) molecules or the single-stranded deoxyribonucleic acid (=DNA) molecules contained in the population (I) are transferred into a population (II) of double-stranded DNA molecules,
b) the population (II) of double-stranded DNA molecules is subjected to a denaturing and subsequent renaturing, so that there is a mixture of homoduplex and heteroduplex DNA molecules,
c) heteroduplices formed in the population (II), in which nucleotides which are uncoupled or incorrectly coupled in accordance with Watson and Crick, are chemically reacted with a carbodiimid compound, and also
d) the resultant carbodiimid conversion products are **characterised**,
**characterised in that** a chromatographic purification process is carried out between each of the steps a to d.

2. A process according to Claim 1,
**characterised in that** the chromatographic purification process is a column or batch process which is carried out by using silicon gel.

3. A process according to one of the preceding Claims,
**characterised in that** the purification step is carried out by using a silica column.

4. A process according to one of the preceding Claims,
**characterised in that** one or more of the following compounds are used as the carbodiimid compound: N-cyclohexyl-N'-(2-morpholinoethyl)-carbodiimid; adipindiimid acid dimethyl ester; pyromellit acid diimd; N,N'-bis(2.6-dimethyl phenyl) perylene-3,4,9,10-tetracarbonic acid diimid; bis-(trimethylsilyl)-carbodiimid; N,N'-ditertiary carbonic acid diimid; N,N'-dicyclohexyl carbodiimid; N,N'-diisopropyl carbodiimid; N-(3-dimethyl amino propyl)-N'-ethyl carbodiimid; N,N'-ditridecyl perylene-3,4,9,10-tetracarbonic acid diimid; pimelic diimid acid methyl ester; octane diimid acid dimethyl ester; 1-ethyl-3-(3-dimethyl-amino propyl carbodiimid

5. A process according to one of the preceding Claims,
**characterised in that** the mixture of homoduplex and heteroduplex DNA molecules is treated with NA-specific enzymes and co-factors for the detection of a missing base pairing.

6. A process according to Claim 1,
**characterised in that** the mixture of homoduplex and heteroduplex DNA molecules is reacted with the carbodiimid compound, preferably with the addition of "mismatch identifying proteins".

7. A process according Claim 1 or 2,
**characterised in that** the population (II) is subjected to one or several amplification step/s prior to step c.

8. A process according to one of Claims 4 to 7,
**characterised in that** in step d the mixture of homoduplex and heteroduplex DNA molecules reacted with the carbodiimid compound is subjected to an extension reaction and the extension products are
**characterised** and quantified.

9. A process according to one of the preceding Claims,
**characterised in that** the extension reaction is carried out without primer.

10. A process according to Claim 8,
**characterised in that** a primer extension reaction is carried out.

11. A process according to one of the preceding Claims,
**characterised in that** an oligonucleotide is used in a modified or unmodified form for the extension reaction.

12. A process according to one of the preceding Claims,
**characterised in that** the primer or the extension products are or become marked.

13. A process according to one of the preceding Claims,
**characterised in that** the extension reaction is carried out repeatedly.

14. A process according to one of the preceding Claims,
**characterised in that** a detection of carbodiimid conversion products is carried out without a prior extension reaction.

15. A process according to one of the preceding Claims,
**characterised in that** the carbodiimid conversion products are identified by means of carbodiimid-specific antibodies.

## Revendications

1. Procédé de détection et de quantification de molécules d'acide nucléique (=AN) au sein d'une population (I) de molécules d'AN de séquence identique, similaire ou déviante, dans lequel
a) les molécules d'acide ribonucléique (=ARN) simple brin ou double brin contenues dans la population (I) ou les molécules d'acide désoxyribonucléique (ADN) simple brin sont transférées dans une population (II) de molécules d'ADN double brin,
b) la population (II) des molécules d'ADN double brin est soumise à une dénaturation puis à une renaturation, de sorte qu'un mélange de molécules d'ADN homo- et hétéroduplex existe,
c) les hétéroduplex formés dans la population (II), dans lesquels existent des nucléotides non appariés ou incorrectement appariés selon Watson et Crick, sont convertis par voie chimique avec un composé de carbodiimide, et
d) les produits de la conversion de carbodiimide formés sont
**caractérisés**,
**caractérisé en ce que**, entre chacune des étapes a à d, on réalise un procédé de purification par chromatographie.

2. Procédé selon la revendication 1, **caractérisé en ce que** le procédé de purification par chromatographie est un procédé en colonne ou par lot, qui est réalisé en utilisant un gel de silice.

3. Procédé selon l'une des revendications précédentes, **caractérisé en ce que** l'étape de purification est réalisée en utilisant une colonne de silice.

4. Procédé selon l'une des revendications précédentes, **caractérisé en ce que** l'on utilise en tant que composé de carbodiimide un ou plusieurs des composés suivants : N-cyclohexyl-N'-(2-morpholinoéthyl)-carbodiimide ; diméthylester d'acide adipine-diimidique ; diimide d'acide pyromellitique ; diimide d'acide N-N'-bis(2,6-diméthylphényl)pérylène-3,4,9,10-tétracarboxylique ; bis-(triméthylsilyl)-carbodiimide ; N-N'-di-butyl tertiaire-carbodiimide ; N,N'-dicyclohexylcarbodiimide ; N,N'-diisopropylcarbodiimide ; N-(3-diméthylaminopropyl)-N'-éthylcarbodiimide ; diimide d'acide N,N'-ditridécylpérylène-3,4,9,10-tétracarboxylique ; diméthylester d'acide pimélinediimidique ; diméthylester d'acide octanediimidique ; 1-éthyl-3-(3-diméthyl-aminopropylcarbodiimide).

5. Procédé selon l'une des revendications précédentes, **caractérisé en ce que** le mélange de molécules d'ADN homo- et hétéroduplex est mélangé pour l'identification d'une paire de bases faisant défaut avec des enzymes spécifiques de l'AN et des cofacteurs.

6. Procédé selon la revendication 1, **caractérisé en ce que** le mélange de molécules d'ADN homo- et hétéroduplex est mis à réagir avec le composé de carbodiimide, de préférence en ajoutant des protéines "d'identification des mauvais appariements".

7. Procédé selon la revendication 1 ou 2, **caractérisé en ce que** la population (II) est soumise avant l'étape c à une ou plusieurs étape(s) d'amplification.

8. Procédé selon l'une des revendications 4 à 7, **caractérisé en ce que** dans l'étape d, le mélange de molécules d'ADN homo- et hétéroduplex converti avec le composé carbodiimide, est soumis à une réaction d'extension, et les produits de l'extension sont **caractérisés** et quantifiés.

9. Procédé selon l'une des revendications précédentes, **caractérisé en ce que** la réaction d'extension est réalisée sans amorce.

10. Procédé selon la revendication 8, **caractérisé en ce que** l'on réalise une réaction d'extension d'amorce.

11. Procédé selon l'une des revendications précédentes, **caractérisé en ce que** l'on utilise pour la réaction d'extension un oligonucléotide sous une forme modifiée ou non modifiée.

12. Procédé selon l'une des revendications précédentes, **caractérisé en ce que** l'amorce ou les produits d'extension sont ou seront marqués.

13. Procédé selon l'une des revendications précédentes, **caractérisé en ce que** la réaction d'extension est réalisée plusieurs fois.

14. Procédé selon l'une des revendications précédentes, **caractérisé en ce qu'**une détection des produits de conversion du carbodiimide est réalisée sans réaction d'extension préalable.

15. Procédé selon l'une des revendications précédentes, **caractérisé en ce que** les produits de conversion du carbodiimide sont identifiés au moyen d'anticorps spécifiques du carbodiimide.
